# EUROPEAN PATENT SPECIFICATION

(11) **EP 4 127 653 B1**
(45) Date of publication and mention of the grant of the patent: **18.11.2026**
(21) Application number: 21713655.5
(22) Date of filing: 19.03.2021
(51) Int. Cl.: G01N 15/1434, G01N 15/00, G01N 15/10, G02B 21/00, G02B 21/34, G02B 21/14

(54) **CHARACTERIZATION OF GENE THERAPY VECTORS**
CHARAKTERISIERUNG VON GENTHERAPIEVEKTOREN
CARACTÉRISATION DE VECTEURS DE THÉRAPIE GÉNIQUE

(30) Priority: 24.03.2020 GB 202004254
(43) Date of publication of application: 08.02.2023
(60) Divisional application: 25151494.9 / 4 524 637
(73) Proprietor: Cytiva BioProcess R&D AB, 751 84 Uppsala (SE)
(72) Inventor: MIELL, Matthew, London Middlesex N14 4LH (GB)
(74) Representative: Spjuth, Nora Sofia
(86) International application number: PCT/EP2021/057103
(87) International publication number: WO 2021/191079

(56) References cited:
- EP-A1- 3 485 309
- US-A1- 2019 004 299
- RAPER VIVIANNE: ""Weighing" for Viral Capsids Wins Best New Technology Award", 5 January 2021 (2021-01-05), XP093112298, Retrieved from the Internet <URL:https://www.genengnews.com/topics/bioprocessing/weighing-for-viral-capsids-wins-best-new-technology-award/> [retrieved on 20231214]
- GOLDFAIN AARON M. ET AL: "Dynamic Measurements of the Position, Orientation, and DNA Content of Individual Unlabeled Bacteriophages", JOURNAL OF PHYSICAL CHEMISTRY PART B, vol. 120, no. 26, 22 April 2016 (2016-04-22), US, pages 6130 - 6138, XP093112302, ISSN: 1520-6106, DOI: 10.1021/acs.jpcb.6b02153
- REFEYN: "Tweet", 18 February 2020 (2020-02-18), XP093112304, Retrieved from the Internet <URL:https://twitter.com/refeynit/status/1229753736844713990?t=z51%207kzH_FAXWpSPBHbfsZA&s=19> [retrieved on 20231214]
- STRACK RITA: "Weighing single proteins with light", NATURE METHODS, NATURE PUB. GROUP, NEW YORK, vol. 15, no. 7, 2 July 2018 (2018-07-02), pages 477, XP036538729, ISSN: 1548-7091, [retrieved on 20180702], DOI: 10.1038/S41592-018-0055-6
- YOUNG GAVIN ET AL: "Interferometric Scattering Microscopy", ANNU. REV. PHYS. CHEM, vol. 70, 1 January 2019 (2019-01-01), pages 301 - 322, XP055812312, Retrieved from the Internet <URL:https://www.annualreviews.org/doi/pdf/10.1146/annurev-physchem-050317-021247> [retrieved on 20210609], DOI: 10.1146/annurev-physchem-050317-
- ELIZABETH E. PIERSON ET AL: "Resolving Adeno-Associated Viral Particle Diversity With Charge Detection Mass Spectrometry", ANALYTICAL CHEMISTRY, vol. 88, no. 13, 5 July 2016 (2016-07-05), US, pages 6718 - 6725, XP055470116, ISSN: 0003-2700, DOI: 10.1021/acs.analchem.6b00883
- SONN-SEGEV ADAR ET AL: "Quantifying the heterogeneity of macromolecular machines by mass photometry", BIORXIV, 4 December 2019 (2019-12-04), XP055812871, Retrieved from the Internet <URL:https://www.biorxiv.org/content/10.1101/864553v1.full.pdf> [retrieved on 20210610], DOI: 10.1101/864553
- WANG CHUNLEI ET AL: "Developing an Anion Exchange Chromatography Assay for Determining Empty and Full Capsid Contents in AAV6.2", MOLECULAR THERAPY- METHODS & CLINICAL DEVELOPMENT, vol. 15, 1 December 2019 (2019-12-01), GB, pages 257 - 263, XP055812692, ISSN: 2329-0501, Retrieved from the Internet <URL:https://www.cell.com/action/showPdf?pii=S2329-0501(19)30104-4> [retrieved on 20210610], DOI: 10.1016/j.omtm.2019.09.006
- WU DI ET AL: "Rapid Characterization of AAV gene therapy vectors by Mass Photometry", BIORXIV, 19 February 2021 (2021-02-19), XP055807769, Retrieved from the Internet <URL:https://www.biorxiv.org/content/10.1101/2021.02.18.431916v1.full.pdf> [retrieved on 20210526], DOI: 10.1101/2021.02.18.431916
- REFEYN: "Mass photometry of AAVs - application note", 1 January 2020 (2020-01-01), XP055812865, Retrieved from the Internet <URL:https://b1cd404c-33b3-4241-83b5-3b96f79f7fca.filesusr.com/ugd/8a9b7f_b30127a4db4d40ee9119920c8ad81029.pdf> [retrieved on 20210610]

## Description

### Technical field of the invention

The present disclosure relates to characterization of gene therapy vectors and viral particles, and more particularly to a method of determining the levels of empty and full capsids in a viral particle preparation.

### Background of the invention

Recombinant viruses show great promise and utility as a vehicle to deliver therapeutic nucleic acids for gene therapy applications. A number of different recombinant viruses are used in these gene therapy applications based on a number of factors including the size of the nucleic acid to be delivered, the target cell or tissue to deliver the nucleic acid, the need for short or long term expression of the therapeutic nucleic acid, and integration of the therapeutic nucleic acid into the recipient's genome. Examples of viruses used in gene therapy applications include adeno-associated virus (AAV), adenovirus, lentivirus and herpes simplex virus (HSV). Also non-viral vectors can be used for delivery of nucleic acids in gene therapy. Examples of such vectors include liposomes and other lipoplexes (see e.g. US2009305409, US6749863, US6071533 and US6303378), polymersomes and polyplexes(see e.g US20110206751, US2017000743 and US2011151013), as well as inorganic and organic nanoparticles.

Polymerase Chain Reaction (PCR) is a common technique for determining the genome content of adeno-associated viruses (AAV), and Analytical Ultracentrifugation (AUC) (see e.g. US2018180525 ) is commonly used for estimating the level of empty capsids (containing only the protein capsids with no DNA) and full capsids (containing both protein and DNA). Also spectrophotometric methods (e.g. WO2019212922) have been used to determine levels of empty and full capsids. Document Young, G. and Kukura, P., Annu. Rev. Phys. Chem, vol 70, 301-322 refers to interferometric scattering microscopy (iSCAT) images of full and empty bacteriophages.

However, these techniques require multiple steps with complicated procedures that are time-consuming and relatively low-throughput. More efficient and better quantitative techniques are thereby desirable for sample screening, in-process sample analysis for process control and monitoring, and final product concentration determination and stability monitoring.

Accordingly, there is a need for simple and rapid methods to characterize virus preparations with respect to empty and full capsids. Likewise, there is a need for simple and rapid methods to characterize non-viral vectors with respect to their being loaded or not with the intended nucleic acid.

### Summary of the invention

The aspect of the invention is to provide a method of distinguishing empty and full capsids in a virus preparation. This is achieved with a method comprising the steps of:
a) providing a preparation of viral particles, wherein the viral particles are adeno-associated virus (AAV) particles;
b) subjecting the preparation to interferometric scattering mass spectrometry (ISCAMS), in an interferometric scattering microscope, to generate mass distribution data for the viral particles;
c) determining the levels of empty capsids and capsids comprising a genome among the viral particles from the mass distribution data.

One advantage is that the method is rapid (a few minutes) and simple and has a high accuracy. A further advantage is that the method is able to measure particles from samples where there is a complex mixture of differently sized particles present; smaller particles are not "hidden" by the presence of larger particles. A yet further advantage is that minimal amounts of sample (<~20 µl) are needed. These advantages make the method a particularly useful technology for at-line process analytical testing (PAT).

Further suitable embodiments of the invention are described in the dependent claims.

### Drawings

Fig. 1 shows the mass distribution for a sample of empty capsids of AAV serotype 5.
Fig. 2 shows the mass distribution for a sample of full capsids of AAV serotype 5.
Fig. 3 shows the mass distribution for a 1:1 mixture of empty and full capsids of AAV serotype 5.
Fig. 4 shows the mass distribution for a 2:1 mixture of empty and full capsids of AAV serotype 5.
Fig. 5 shows mass distributions of full AAV serotype 5 capsids:
   a) in PBS buffer + 130 mM NaCl, 0.001% (v/v) Pluronic F-188
   b) in PBS buffer + 130 mM NaCl, 0.001% (v/v) Pluronic F-188 (replicate)
   c) in 20 mM TRIS buffer pH 8.5, no salt
   d) in 20 mM TRIS buffer pH 8.5, no salt (replicate)
Fig. 6 shows mass distributions of full AAV serotype 5 capsids in a preparation diluted 1000x.

### Definitions

As used herein, the terms "comprises," "comprising," "containing," "having" and the like can have the meaning ascribed to them in U.S. Patent law and can mean "includes," "including," and the like; "consisting essentially of or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

### Detailed description of embodiments

In one aspect, the present invention discloses a method of characterizing a preparation of viral particles comprising the steps of:
a) Providing a preparation of adeno-associcated viral particles. The preparation can e.g. be a preparation of viral particles intended to be used as viral vectors for cell/gene therapy but they can also be intended to be used e.g. as a vaccine antigen. The viral particles can suitably be recombinant, with an engineered genome in the capsids and the preparation may also include empty capsids. The preparation may comprise at least 5x10⁸ viral particles per ml preparation, such as at least 1x10⁹ viral particles per ml preparation, 5x10⁸ - 1x10¹³ or 1x10⁹ - 1x10¹² viral particles per ml preparation. The preparation can suitably comprise a buffer, e.g. a PBS or Tris buffer, which may have a pH of 3-9, such as 6-8.5 or 7-8.5, and which may further comprise a salt, e.g. NaCl. Further, the preparation may comprise a dispersant, such as a poloxamer (ethylene oxide-propylene oxide block copolymers). Examples of poloxamer dispersants can be poloxamer 188 (Cas No. 9003-11-6), e.g. Pluronic F-188 (BASF) or Pluronic F-68. The preparation can typically be a cell culture fluid (e.g. a cell lysate), a partially purified cell culture fluid/cell lysate or a purified suspension of viral particles originating from a cell culture fluid/cell lysate.
b) Subjecting the preparation to interferometric scattering mass spectrometry (ISCAMS), in an interferometric scattering microscope, to generate mass distribution data for the viral particles. In this step, the intensity of scattered light from individual virus particles on a surface can be measured and the mass distribution data calculated from a scattered light intensity distribution. The interferometric scattering microscope can be constructed as disclosed in US20190004299, and it can in particular be a Refeyn OneMP microscope, commercially available from Refeyn Ltd., Oxford, UK. In this instrument a sample of the preparation is placed onto a glass slide (which may be functionalized to bind or exclude certain biomolecules) and the slide placed above the lens of the microscope. The instrument uses laser light focused on the surface of the slide and the point-spread function of molecules/particles are observed as they come close to the surface. The equipment can distinguish particles as they bind and unbind from the surface, as well as "wobbling" across the surface. To clearly distinguish these particles the software divides consecutive frames of the movie to provide "ratiometric imaging". The microscope detects the interference between the reflected and scattered light from single molecules/particles. The intensity, or contrast, of this interference point then correlates to the density of the molecule. The density of proteins remains constant over quite a range, as does the density of DNA, and this scales with particle mass. So, with the help of calibration reference standards the contrast of the spots can be used to calculate the mass of the particles. The surface holding the virus particles can suitably be a glass surface, such as a glass slide or coverslip. It can be a non-modified glass surface or it can be chemically modified, e.g. with functional silanes. The chemical modification can also involve derivatization with ligands capable of binding the viral particles, e.g. llama antibody ligands that have been shown to bind AAV of serotypes 1, 2, 3, 4 and 5 (as used in AVB Sepharose High Performance, GE Healthcare). With regard to the construction of the microscope, it can typically comprise:
   a sample holder for holding a sample in a sample location;
   an illumination source arranged to provide illuminating light;
   a detector;
   an optical system arranged to direct illuminating light onto the sample location and further arranged to collect output light in reflection, the output light comprising both light scattered from the sample location and illuminating light reflected from the sample location, and yet further arranged to direct the output light to the detector; and
   a spatial filter positioned to filter the output light, the spatial filter being arranged to pass output light but with a reduction in intensity that is greater within a predetermined numerical aperture than at larger numerical apertures. The sample holder can suitably comprise a surface (as discussed above) and viral particles the interact with the surface.
c) Determining the levels of empty capsids and capsids comprising a genome (full capsids) among the viral particles from the mass distribution data. The empty and full capsids show up as distinct peaks in the mass distribution and the relative numbers can be obtained by integrating the peaks or adding the counts in each peak when presented as a histogram. The viral particles are much larger than essentially all particles within typical cell lysates, so the viral particle peaks can clearly be distinguished from other cellular protein / DNA contaminants.

In some embodiments, the method is performed for at-line testing in a process for manufacturing viral vectors. It can suitably be used in process analytical technology (PAT), e.g. wherein at least one process parameter is adjusted when a predetermined ratio of empty capsids to full capsids is reached. In this case, the samples may be complex, i.e. with many other biomolecules present. In particular, the method can be applied in conjunction with a chromatography step, e.g. where the method is performed on an eluate or a flowthrough from a chromatography step. The process parameter can then e.g. be the amount of a viral vector preparation loaded on a chromatography column or device. If the ratio of empty to full capsids is too high in a chromatographic cycle, the amount loaded can be decreased in a subsequent cycle. To increase the selectivity for the viral vectors, e.g. AAV vectors, it can be advantageous to use surfaces derivatized with ligands that selectively bind the vectors. It can also be advantageous to use diluted samples to reduce the total biomolecule concentration.

Further disclosed, but not being part of the invention, is a method of distinguishing non-viral gene therapy vectors loaded with a nucleic acid from vectors which are not loaded with a nucleic acid, comprising the steps of:
a) Providing a preparation of non-viral gene therapy vectors. The vectors can e.g. be liposomes or other lipoplexes, polymersomes or polyplexes, as well as inorganic and organic nanoparticles. The vectors can suitably have diameters below 1000 nm, such as below 500 nm, below 200 nm or below 100 nm. They can e.g. have volume-weighted average diameters of 10-1000 nm, such as 10-500 nm, 10-200 nm, 10-100 nm, 20-500 nm, 20-500 nm or 20-100 nm.
b) Subjecting the preparation to interferometric scattering mass spectrometry (ISCAMS), in an interferometric scattering microscope as discussed above, to generate mass distribution data for the vectors. As discussed above, the intensity of scattered light from individual vectors present on a surface may be measured and the mass distribution data are calculated from a scattered light intensity distribution. The vectors can be present on a glass surface, such as a glass slide or coverslip surface. This glass surface may or may not be chemically modified, in particular with ligands capable of binding the vectors. In case the vectors have diameters above 20 nm, such as above 50 nm or above 100 nm, the interferometric scattering microscope may be fitted with a high wavelength laser, such as a laser emitting light of above 600 nm, such as above 700 nm, 600-1000 nm, 600-800 nm or 700-800 nm.
c) Determining the levels of vectors loaded with a nucleic acid and vectors which are not loaded with nucleic acid from the mass distribution data.

### Examples

The experiments were carried out with a Refeyn OneMP microscope, where a sample was placed on a non-functionalized glass slide above the microscope lens. The ISCAMS analysis was carried out according to the microscope instructions and the data were plotted as particle counts vs. particle mass histograms. For the peaks identified, average mass, standard deviation and total peak count were calculated.

We tested purified AAV-5 samples using non-functionalized glass slides. In our setup it was not possible to determine absolute counts of the AAV titer, this would require functionalizing the surface such that AAV particles are only counted once during the sampling time. This is possible using existing or new affinity ligands against AAV, but was not explicitly tested here.

The detection method enables mass photometry to measure the mass of the AAV capsids, and so distinguish empty and full AAV virions. This is achieved without labelling and, as the AAV are much larger than almost all particles within typical cell lysates, the AAV can clearly be distinguished from other cellular protein / DNA contaminants. We found AAV particles generally did not bind to the glass surface, so could be counted many times. This was of benefit as long-movie collection times could be used to ensure a high number of particle counts. However, whilst this method provides a ratiometric comparison for empty:full particles it does not provide an absolute count of AAV titres. To achieve that would require AAV particles bind and stay bound to the imaging surface, which is certainly possible if the surface were functionalized with an affinity ligand or similar ligand to enable capture of the AAV.

### Example 1

Two purified preparations of AAV serotype 5 viral particles were used - one with only empty capsids and one with only full capsids. In both cases, the viral particles were suspended in a PBS buffer (pH 7.4) with 130 mM NaCl and 0.001% (v/v) Pluronic F-188. The virions were purchased from Vigene Biosciences (Maryland, USA). The AAV was purified and separated into empty:full by density ultracentrifugation, then diafiltered into the buffer (PBS+130mM NaCl with Pluronic F-188). The concentration of full AAV-5 particles was initially 2.95x10¹³ capsids/ml, and empty capsids were 1.61x10¹³ capsids/ml. Both the empty and full were then diluted to ~1x10¹² capsids/ml in the PBS+ 130mM NaCl buffer.

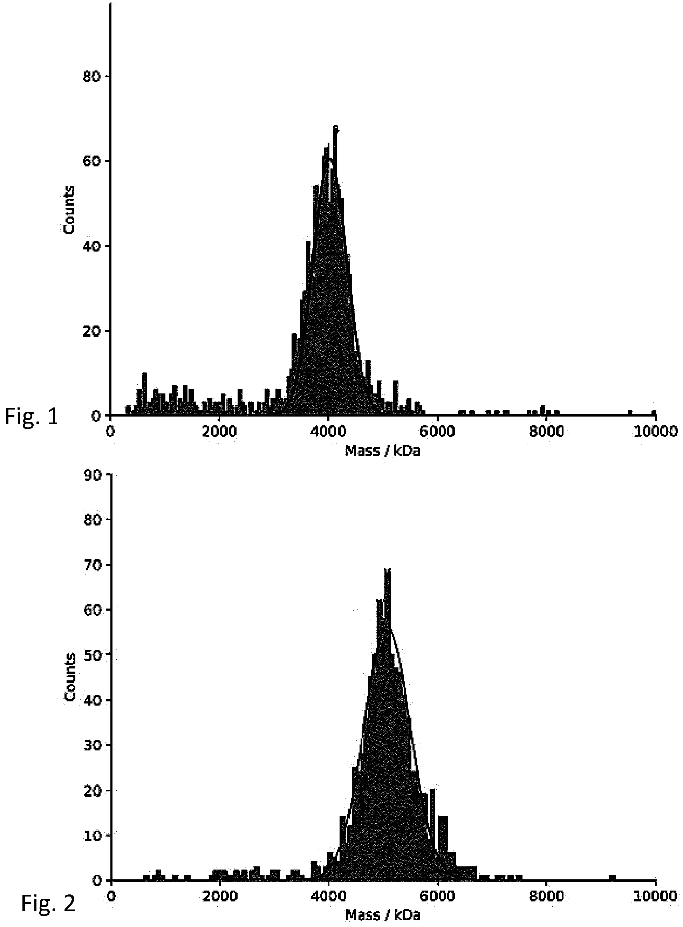

As shown in Figs. 1 and 2, the empty capsids formed a peak at 4 000 kDa and the full capsids formed a peak at 5 000 kDa. The peak parameters are shown in Table 1.

**Table 1. Data for empty and full AAV-5 capsids**

| **Sample** | **Average mass, kDa** | **Standard deviation, kDa** | **Counts** |
|---|---|---|---|
| AAV-5 empty capsids | 4024 | 309 | 938 |
| AAV-5 full capsids | 5064 | 428 | 804 |

### Example 2

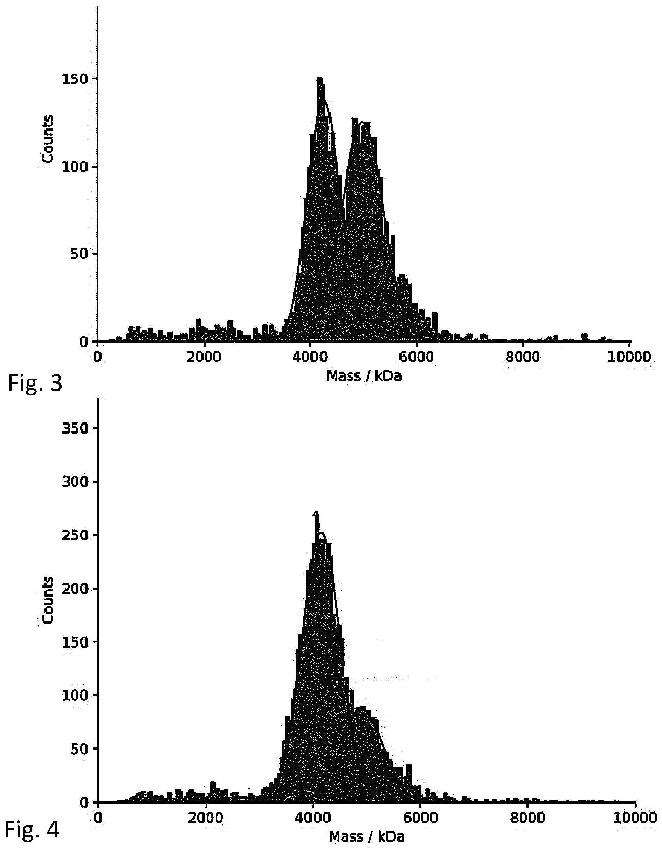

The same AAV-5 preparations as in Example 1 were mixed to give i) an empty-to-full capsid ratio of 1:1 and ii) a ratio of 2:1. The results as shown in Figs. 3 and 4 indicate that the two peaks can be resolved by curve-fitting techniques, assuming normal distributions. The resolved peak data are shown in Table 2.

**Table 2. Data for mixtures of empty and full AAV-5 capsids**

| **Sample** | **Average mass, kDa** | | **Standard deviation, kDa** | | **Counts** | |
|---|---|---|---|---|---|---|
| | **Empty** | **Full** | **Empty** | **Full** | **Empty** | **Full** |
| Empty:full 1:1 | 4250 | 4975 | 303 | 389 | 1738 | 2036 |
| Empty:full 2:1 | 4146 | 4910 | 352 | 393 | 4451 | 1704 |

### Example 3

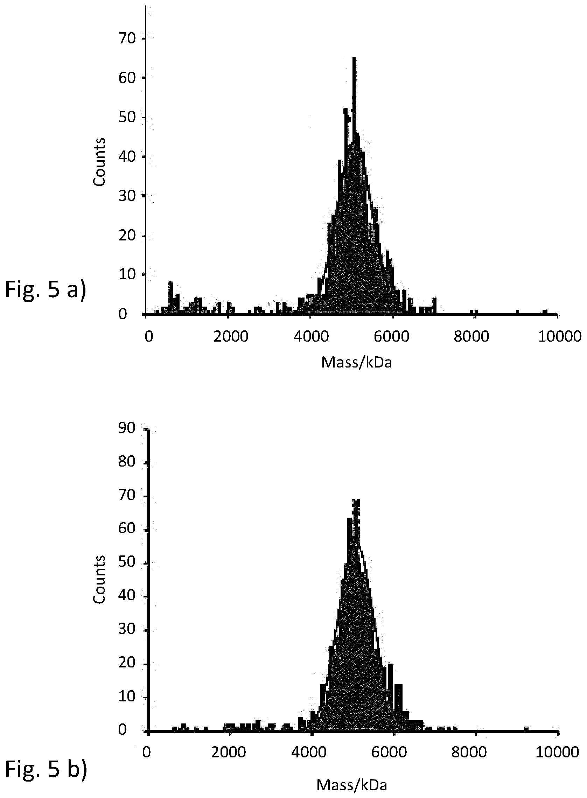

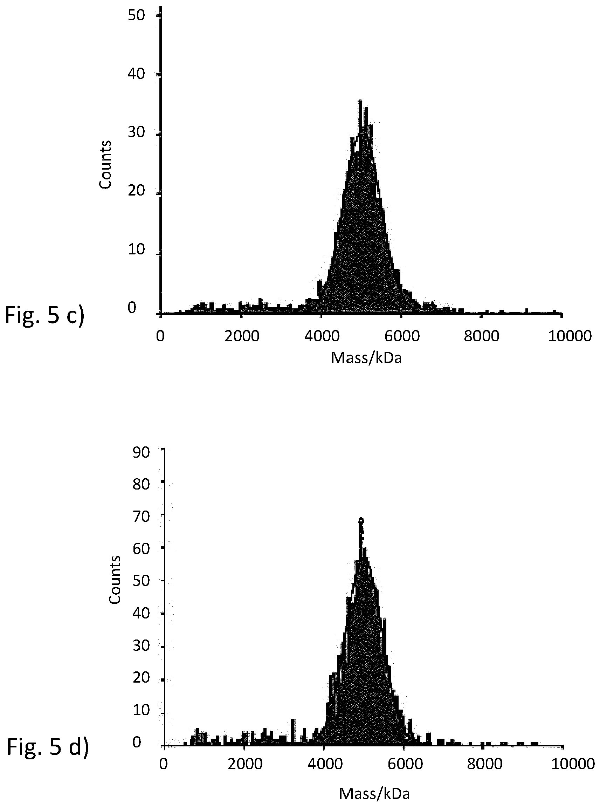

Two AAV-5 preparations were used - one in the PBS, 130 mM NaCl, 0.001% Pluronic F-188 buffer and one in a Tris buffer pH 8.5 with no salt added. Two replicate samples were analyzed for each preparation. The results are shown in Fig. 5 a) - d) and Table 3, and demonstrate that the mass distribution data were repeatable and that no difference between the two buffers could be observed.

**Table 3. Data for full AAV-5 capsids in different buffers**

| **Sample** | **Average mass, kDa** | **Standard deviation, kDa** | **Counts** |
|---|---|---|---|
| PBS, 130 mM NaCl, Pluronic | 5077 | 422 | 929 |
| | 5064 | 428 | 804 |
| Tris pH 8.5 | 5021 | 463 | 3559 |
| | 5003 | 454 | 1294 |

### Example 4

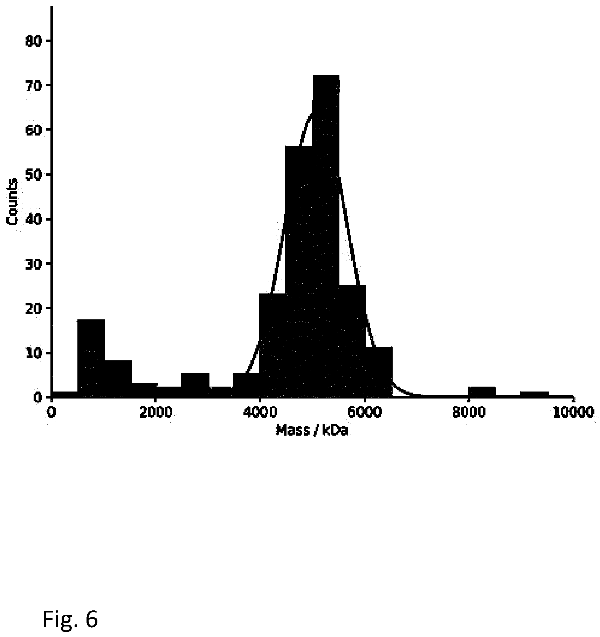

The same AAV-5 preparation as in Example 1 was diluted 1000 times with the buffer (i.e. to a final concentration of ~1x10⁹ capsids/mL) and analyzed in the microscope. As shown in Fig. 6 and Table 4, although the sampling time was longer, the number of counts was lower. However, the peak average and width data were still consistent.

**Table 4. Data for full AAV-5 capsids diluted 1000 times in PBS buffer.**

| **Average mass, kDa** | **Standard deviation, kDa** | **Counts** |
|---|---|---|
| 5067 | 596 | 191 |

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A method of characterizing a preparation of viral particles comprising the steps of:
a) providing a preparation of viral particles, wherein the viral particles are adeno-associated virus (AAV) particles;
b) subjecting the preparation to interferometric scattering mass spectrometry (ISCAMS), in an interferometric scattering microscope, to generate mass distribution data for the viral particles;
c) determining the levels of empty capsids and capsids comprising a genome among the viral particles from the mass distribution data.

2. The method of claim 1, wherein in step b), the intensity of scattered light from individual virus particles present on a surface is measured and the mass distribution data are calculated from a scattered light intensity distribution.

3. The method of claim 1 or 2, wherein in step b), viral particles are present on a glass surface, such as a glass slide or coverslip surface, optionally wherein the glass surface is derivatized with ligands capable of binding the viral particles.

4. The method of any preceding claim, wherein said preparation comprises at least 5x10⁸ viral particles per ml preparation.

5. The method of any preceding claim, wherein said preparation comprises a buffer, such as a PBS or Tris buffer, and/or a salt, such as NaCl, and/or a dispersant, e.g. a poloxamer, such as poloxamer 188.

6. The method of any preceding claim, wherein said viral particles are viral vector particles.

7. The method of any preceding claim, wherein said interferometric scattering microscope comprises:
a sample holder for holding a sample in a sample location;
an illumination source arranged to provide illuminating light;
a detector;
an optical system arranged to direct illuminating light onto the sample location and further arranged to collect output light in reflection, the output light comprising both light scattered from the sample location and illuminating light reflected from the sample location, and yet further arranged to direct the output light to the detector; and
a spatial filter positioned to filter the output light, the spatial filter being arranged to pass output light but with a reduction in intensity that is greater within a predetermined numerical aperture than at larger numerical apertures.

8. The method of claim 7, wherein the sample holder comprises a surface and viral particles are present on said surface.

9. The method of claim 8, wherein said surface is a glass surface, such as a glass slide or coverslip surface.

10. The method of claim 8 or 9, wherein said surface is a surface of a microfluidic channel or a multiwell plate.

11. The method of any preceding claim, which is performed as at-line testing in a process for manufacturing or using viral vectors.

12. The method of claim 11, wherein at least one process parameter is adjusted when a predetermined ratio of empty capsids to full capsids is reached.

13. The method of claim 11 or 12, wherein the method is performed on an eluate or a flowthrough from a chromatography step.

## Patentansprüche

1. Verfahren zum Charakterisieren eines Präparats von viralen Partikeln, umfassend die folgenden Schritte:
a) Bereitstellen eines Präparats von viralen Partikeln, wobei es sich bei den viralen Partikeln um Partikel des Adeno-assoziierten Virus (AAV) handelt;
b) Unterziehen des Präparats einer interferometrischen Streumassenspektrometrie (ISCAMS) in einem interferometrischen Streumikroskop zum Erzeugen von Massenverteilungsdaten für die viralen Partikel;
c) Bestimmen des Gehalts von leeren Kapsiden und Kapsiden, die ein Genom umfassen, unter den viralen Partikeln aus den Massenverteilungsdaten.

2. Verfahren nach Anspruch 1, wobei in Schritt b) die Intensität von Streulicht aus einzelnen Viruspartikeln, die auf einer Fläche vorhanden sind, gemessen wird und die Massenverteilungsdaten aus einer Intensitätsverteilung des Streulichts berechnet werden.

3. Verfahren nach Anspruch 1 oder 2, wobei in Schritt b) die viralen Partikel auf einer Glasfläche vorhanden sind, beispielsweise einem Glasobjektträger oder einer Deckglasfläche, wobei optional die Glasfläche mit Liganden derivatisiert ist, welche die viralen Partikel binden können.

4. Verfahren nach einem vorstehenden Anspruch, wobei das Präparat mindestens 5x10⁸ virale Partikel pro ml des Präparats umfasst.

5. Verfahren nach einem vorstehenden Anspruch, wobei das Präparat einen Puffer umfasst, beispielsweise PBS oder Tris-Puffer, und/oder ein Salz, beispielsweise NaCl, und/oder ein Dispergiermittel, z. B. ein Poloxamer, beispielsweise Poloxamer 188.

6. Verfahren nach einem vorstehenden Anspruch, wobei es sich bei den viralen Partikeln um virale Vektorpartikel handelt.

7. Verfahren nach einem vorstehenden Anspruch, wobei das interferometrische Streumikroskop umfasst:
einen Probenhalter zum Halten einer Probe in einer Probenposition;
eine Beleuchtungsquelle, angeordnet zum Bereitstellen von Beleuchtungslicht;
einen Detektor;
ein optisches System, angeordnet zum Richten von Beleuchtungslicht auf die Probenposition und weiter angeordnet zum Sammeln von Ausgabelicht in Reflexion, wobei das Ausgabelicht sowohl von der Probenposition gestreutes Licht als auch von der Probenposition reflektiertes Beleuchtungslicht umfasst, und noch weiter angeordnet zum Richten des Ausgabelichts auf den Detektor; und
ein räumliches Filter, positioniert zum Filtern des Ausgabelichts, wobei das räumliche Filter dafür angeordnet ist, Ausgabelicht hindurch zu lassen, doch mit einer Intensitätsverringerung, die in einer vorbestimmten numerischen Apertur größer ist als bei größeren numerischen Aperturen.

8. Verfahren nach Anspruch 7, wobei der Probenhalter eine Fläche umfasst und die viralen Partikel auf der Fläche vorhanden sind.

9. Verfahren nach Anspruch 8, wobei die Fläche eine Glasfläche ist, beispielsweise ein Glasobjektträger oder eine Deckglasfläche.

10. Verfahren nach Anspruch 8 oder 9, wobei die Fläche eine Fläche eines mikrofluidischen Kanals oder eine Multiwell-Platte ist.

11. Verfahren nach einem vorstehenden Anspruch, das als eine At-Line-Prüfung in einem Prozess zur Herstellung oder Verwendung viraler Vektoren durchgeführt wird.

12. Verfahren nach Anspruch 11, wobei mindestens ein Prozessparameter angepasst wird, wenn ein vorbestimmtes Verhältnis von leeren Kapsiden zu vollen Kapsiden erreicht ist.

13. Verfahren nach Anspruch 11 oder 12, wobei das Verfahren an einem Eluat oder einem Durchfluss aus einem Chromatographieschritt durchgeführt wird.

## Revendications

1. Procédé de caractérisation d'une préparation de particules virales comprenant les étapes de :
a) fourniture d'une préparation de particules virales, dans lequel les particules virales sont des particules de virus adéno-associé (AAV) ;
b) soumission de la préparation à une spectrométrie de masse à diffusion interférométrique (ISCAMS), dans un microscope à diffusion interférométrique, pour générer des données de distribution de masse pour les particules virales ;
c) détermination des niveaux de capsides vides et de capsides comprenant un génome parmi les particules virales à partir des données de distribution de masse.

2. Procédé selon la revendication 1, dans lequel dans l'étape b), l'intensité de la lumière diffusée provenant de particules virales individuelles présentes sur une surface est mesurée et les données de distribution de masse sont calculées à partir d'une distribution d'intensité de lumière diffusée.

3. Procédé selon la revendication 1 ou la revendication 2, dans lequel dans l'étape b), des particules virales sont présentes sur une surface de verre, telle qu'une surface de lame de verre ou de lamelle protectrice, facultativement dans lequel la surface de verre fait l'objet d'une dérivatisation avec des ligands aptes à se lier aux particules virales.

4. Procédé selon une quelconque revendication précédente, dans lequel ladite préparation comprend au moins 5x10⁸ particules virales par ml de préparation.

5. Procédé selon une quelconque revendication précédente, dans lequel ladite préparation comprend un tampon, tel qu'un tampon PBS ou Tris, et/ou un sel, tel que du NaCl, et/ou un dispersant, par exemple un poloxamère, tel que le poloxamère 188.

6. Procédé selon une quelconque revendication précédente, dans lequel lesdites particules virales sont des particules de vecteur viral.

7. Procédé selon une quelconque revendication précédente, dans lequel ledit microscope à diffusion interférométrique comprend :
un porte-échantillon pour maintenir un échantillon dans un emplacement d'échantillon ;
une source d'éclairage agencée pour fournir une lumière d'éclairage ;
un détecteur ;
un système optique agencé pour diriger une lumière d'éclairage sur l'emplacement d'échantillon et en outre agencé pour collecter une lumière de sortie en réflexion, la lumière de sortie comprenant à la fois une lumière diffusée à partir de l'emplacement d'échantillon et une lumière d'éclairage réfléchie à partir de l'emplacement d'échantillon, et en outre agencé pour diriger la lumière de sortie vers le détecteur ; et
un filtre spatial positionné pour filtrer la lumière de sortie, le filtre spatial étant agencé pour laisser passer la lumière de sortie mais avec une réduction d'intensité qui est plus grande dans une ouverture numérique prédéterminée qu'au niveau d'ouvertures numériques plus grandes.

8. Procédé selon la revendication 7, dans lequel le porte-échantillon comprend une surface et des particules virales sont présentes sur ladite surface.

9. Procédé selon la revendication 8, dans lequel ladite surface est une surface de verre, telle qu'une surface de lame de verre ou de lamelle protectrice.

10. Procédé selon la revendication 8 ou la revendication 9, dans lequel ladite surface est une surface d'un canal microfluidique ou d'une plaque multipuits.

11. Procédé selon une quelconque revendication précédente, qui est mis en œuvre sous forme d'essai en ligne dans un processus de fabrication ou d'utilisation de vecteurs viraux.

12. Procédé selon la revendication 11, dans lequel au moins un paramètre de processus est ajusté lorsqu'un rapport prédéterminé des capsides vides aux capsides pleines est atteint.

13. Procédé selon la revendication 11 ou la revendication 12, dans lequel le procédé est mis en œuvre sur un éluat ou un produit d'écoulement provenant d'une étape de chromatographie.
